# FASCICULE DE BREVET EUROPEEN

(11) **EP 1 973 592 B1**
(45) Date de publication et mention de la délivrance du brevet: **21.06.2017**
(21) Numéro de dépôt: 07717969.5
(22) Date de dépôt: 19.01.2007
(51) Int. Cl.: A61M 5/34, A61M 5/31, A61M 5/50

(54) **SERINGUE HYPODERMIQUE PREREMPLIE EQUIPEE D'UN DISPOSITIF DE BOUCHAGE**
VORGEFÜLLTE HYPODERMISCHE SPRITZE MIT EINER VERSCHLUSSVORRICHTUNG
PRE-FILLED HYPODERMIC SYRINGE FITTED WITH A STOPPERING DEVICE

(30) Priorité: 19.01.2006 FR 0600490
(43) Date de publication de la demande: 01.10.2008
(73) Titulaire: Laboratoire Aguettant, 69353 Lyon Cedex 07 (FR)
(72) Inventeur: FREZZA, Pierre, 69390 Charly (FR)
(74) Mandataire: Verriest, Philippe
(86) Numéro de dépôt international: PCT/FR2007/000107
(87) Numéro de publication internationale: WO 2007/083034

(56) Documents cités:
- EP-A- 0 815 884
- EP-A- 1 166 742
- WO-A-2005/123159
- GB-A- 2 006 712
- NL-C2- 1 021 236
- US-A1- 2005 283 116

## Description

L'invention concerne une seringue hypodermique préremplie équipée d'un dispositif de bouchage.

Les seringues préremplies se présentent sous la forme d'une seringue standard dont l'extrémité de sortie comprend un embout équipé d'un bouchon amovible monté de manière étanche sur celui-ci.

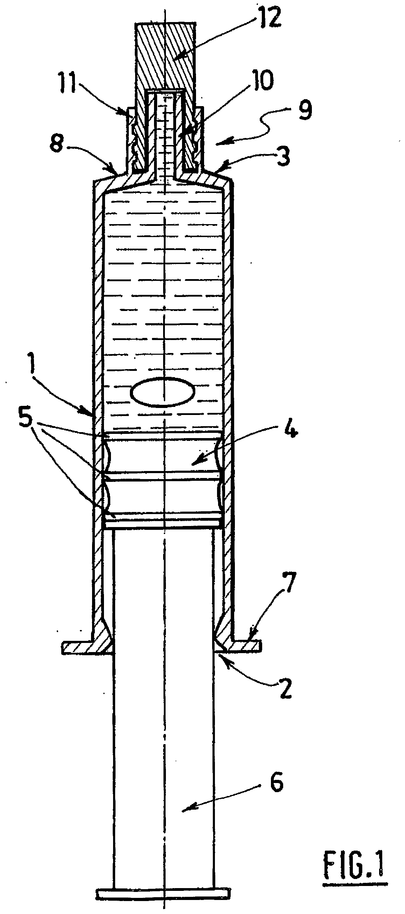

La figure 1 décrit une telle seringue hypodermique préremplie. Celle-ci comporte un corps tubulaire 1 de forme générale cylindrique. Ce corps tubulaire comporte une première extrémité 2 ouverte.

Un poussoir 6 traverse l'ouverture de la première extrémité 2 du corps tubulaire 1. Le poussoir 6 comporte à son extrémité montée dans le corps tubulaire 1, un piston 4 capable de coulisser à l'intérieur du corps tubulaire 1 suivant l'axe longitudinal de celui-ci. Le poussoir 6 permet ainsi à l'utilisateur d'actionner le mouvement de translation du piston.

La surface externe du corps tubulaire 1 comporte une collerette d'appui 7 au niveau de la première extrémité 2. Les doigts de l'utilisateur venant s'appuyer sur la collerette, le piston 4 peut être actionné en pressant sur l'extrémité du poussoir 6 opposée à celle reliée au piston 4.

La seconde extrémité 3 du corps tubulaire comporte une paroi 8 sensiblement transversale et ouverte en son centre.

Le corps tubulaire 1 présente en outre un embout de raccordement 9 de type Luer-lock, disposé au niveau de sa seconde extrémité 3, sur la face externe de la paroi 8. Comme cela est connu en soi, l'embout 9 est composé d'une partie tubulaire 10 et d'une partie filetée 11 formant un col.

La partie tubulaire 10 est de forme sensiblement conique, l'ouverture située du côté de plus grand diamètre de l'embout coïncidant avec l'ouverture de la paroi 8.

La partie filetée 11 de l'embout est destinée au vissage d'un embout de forme complémentaire permettant de raccorder une aiguille ou une ligne de perfusion à la seringue. La partie filetée 11 est disposée coaxialement à la partie tubulaire 10 et à l'extérieur de celle-ci.

Un bouchon 12 est vissé sur l'embout 9, au moyen de la partie filetée 11, recouvrant ainsi l'ouverture de la partie tubulaire 10 de l'embout 9.

Le corps tubulaire 1, le piston 4, la paroi 8, la partie tubulaire 10 de l'embout 9 et le bouchon 12 délimitent ainsi un espace étanche servant de réservoir à un fluide à administrer à un patient.

Le bouchon 12 obturant la partie tubulaire 10 de l'embout 9 peut être retiré par dévissage. Une fois l'aguille ou la ligne de perfusion raccordée à l'embout 9, le poussoir 6 permet à l'opérateur d'actionner le piston 4 et d'administrer ainsi au patient le fluide contenu dans le réservoir.

L'avantage d'une seringue préremplie est d'éviter à l'utilisateur de transférer un médicament d'un contenant quelconque dans la seringue avant utilisation. Ces seringues comportent généralement une graduation permettant le dosage désiré.

La seringue préremplie est conditionnée dans un blister fermé par un opercule en papier pelable. Ce papier possède la particularité d'être perméable à la vapeur d'eau mais imperméable aux micro-organismes. La stérilisation de l'ensemble est réalisée en chaleur humide, c'est-à-dire par contact des parties à stériliser avec de la vapeur à haute température. Cette vapeur provient d'une enceinte d'un autoclave, traverse l'opercule pelable et accède ainsi aux différentes parties à stériliser de la seringue.

Toutefois, le bouchon amovible étant monté de manière étanche sur l'embout, la zone comprise entre ces deux éléments n'est pas accessible à la vapeur. La face externe de l'embout ne peut donc pas être stérilisée en utilisant le procédé de stérilisation en chaleur humide.

L'ensemble doit alors être stérilisé en chaleur sèche de manière à garantir la stérilité entre le bouchon et l'embout sur lequel il est monté.

Ce procédé nécessite une durée de stérilisation plus longue que la stérilisation en chaleur humide.

L'allongement de la durée de stérilisation augmente le coût de production d'une telle seringue. Il entraîne également une dégradation plus importante de la matière plastique du corps de la seringue préremplie, augmentant ainsi le risque de relargage de produits de dégradation dans le soluté. En outre, l'exposition tardive à de hautes températures engendre une dégradation rédhibitoire de certains principes actifs.

Un problème supplémentaire provient de la fermeture de la seringue par un bouchon.

Dans le cas de l'utilisation d'un bouchon en matière plastique, généralement en polypropylène, celui-ci est habituellement vissé sur un embout du type Luer-Lock.

Quel que soit le couple de serrage au moment du vissage du bouchon sur l'embout, on observe un relâchement du serrage pendant la stérilisation. Ce relâchement est causé par le ramollissement du polypropylène à haute température, rendant aléatoire l'étanchéité entre le bouchon et l'embout.

Dans le cas de l'utilisation d'un bouchon en élastomère monté par serrage élastique sur l'embout, le bouchon n'est pas affecté par l'élévation de température. Toutefois, la stérilisation crée une adhérence du bouchon sur l'embout, rendant difficile l'enlèvement du bouchon par l'utilisateur.

Le document US 2005/0283116 décrit une seringue hypodermique préremplie comportant :
- un corps tubulaire comprenant deux extrémités ouvertes et contenant un fluide à administrer à un patient,
- un poussoir qui, comportant à l'une de ses extrémités un piston monté coulissant dans le corps tubulaire, traverse l'ouverture de la première extrémité du corps tubulaire,
- un embout de raccordement en forme de cône du type Luer ou Luer-Lock, par exemple à une aiguille ou à une ligne de perfusion, destiné au passage du fluide à administrer et comportant une partie tubulaire communiquant avec l'ouverture de la seconde extrémité,
l'embout comportant un obturateur relié par une zone sécable à l'extrémité libre de sa partie tubulaire,
la seringue étant réalisée en matière synthétique et obtenue par moulage, l'obturateur venant de moulage en une seule pièce avec l'embout et le corps tubulaire,
la seringue comportant un capuchon monté sur l'embout.

La seringue est obtenue par moulage, les formes externes de la seringue étant délimitée, de manière classique, par les parois d'un moule, un noyau délimitant le volume interne du corps tubulaire et de la partie tubulaire de l'embout. Afin de compenser les tolérances de dimensionnement et les écarts de positionnement du noyau dans le moule, la zone sécable ménagée à l'extrémité de la partie tubulaire de l'embout doit nécessairement comporter une épaisseur suffisante pour assurer une continuité de la matière, même en cas de défaut de positionnement du noyau.

Le retrait de l'obturateur est effectué en faisant basculer latéralement ce dernier à l'aide d'un capuchon muni d'une fente.

Dans ce cas, la zone sécable reliant l'obturateur à l'embout est soumise à des contraintes hétérogènes, à savoir des contraintes de traction d'un côté et des contraintes de compression de l'autre côté. La mauvaise répartition des contraintes dans la zone sécable rend incertaine la rupture complète de la zone sécable par un seul mouvement de basculement, obligeant généralement l'utilisateur à effectuer un mouvement alternatif. La présence d'une épaisseur de matière au niveau de la zone sécable rend d'autant plus difficile la séparation nette de l'obturateur et de l'embout.

Si une cassure nette n'est pas obtenue, des particules peuvent être produites lors de l'arrachage de l'embout de sorte que la solution contenue dans la seringue est polluée.

Le document EP1166742 divulgue une seringue hypodermique préremplie selon le préambule de la revendication 1.

La présente invention vise donc à remédier à ces inconvénients en proposant une seringue pouvant être stérilisée facilement et sans surcoût, dont le contenu n'est pas affecté par la stérilisation et offrant une étanchéité parfaite, tout en permettant de séparer efficacement l'obturateur de l'embout.

A cet effet l'invention concerne une seringue hypodermique préremplie selon la revendication 1.

Le couple exercé sur l'obturateur lors de la rotation de celui-ci autour de l'axe longitudinal entraîne une répartition homogène des contraintes au niveau de la zone sécable.

Le sectionnement est donc obtenu avec un effort limité et de façon nette, c'est-à-dire sans production de particules parasites ou formation d'irrégularités au niveau de la zone sécable.

En outre, ce type de seringue permet de s'affranchir facilement des écarts de dimensions ou des tolérances engendrées lors de la fabrication. En effet, une dissymétrie de la zone sécable a peu d'effet sur le sectionnement de celle-ci, du fait de la bonne répartition des contraintes.

Avantageusement, la liaison entre la partie tubulaire de l'embout et l'obturateur est réalisée par un amincissement annulaire de la matière le long de la ligne de raccordement entre l'extrémité libre de la partie tubulaire de l'embout et l'obturateur.

Selon un premier mode d'exécution, le capuchon comporte une paroi d'extrémité à partir de laquelle fait saillie une cheminée qui, engagée sur l'obturateur, coopère avec celui-ci par complémentarité de forme de manière à réaliser un couplage en rotation.

Préférentiellement, l'obturateur et la cheminée présentent des formes polygonales complémentaires, par exemple hexagonales.

Avantageusement, l'obturateur et/ou la cheminée présentent une forme conique.

Cette caractéristique permet l'emmanchement à force de l'obturateur dans la cheminée de sorte que, après rupture de la zone sécable, l'obturateur soit maintenu dans la cheminée et ne puisse pas tomber au sol.

Selon un second mode d'exécution de l'invention, le capuchon est fixé sur l'obturateur, notamment par soudage ou par collage.

Les deux modes d'exécution précités permettent de séparer l'obturateur de l'embout par l'intermédiaire d'un capuchon protecteur. De ce fait, l'obturateur peut être retiré par l'utilisateur sans risque de contact avec l'élément tubulaire de l'embout, évitant ainsi toute contamination bactérienne du fluide transitant vers l'aiguille ou la ligne de perfusion par l'intermédiaire de l'élément tubulaire de l'embout.

Selon une caractéristique de l'invention, le capuchon comporte des moyens ergonomiques de mise en rotation permettant de faciliter la rotation du capuchon et donc l'ouverture de la seringue par retrait de l'obturateur.

L'orifice du capuchon permet à la vapeur d'accéder facilement aux parties à stériliser, recouvertes en partie ou en totalité par le capuchon. C'est ainsi que lors de la stérilisation en chaleur humide, la vapeur provenant de l'autoclave traverse l'orifice du capuchon et se dirige notamment vers les surfaces externes de l'embout.

La ceinture d'inviolabilité permet de garantir à l'utilisateur la stérilité des parties de la seringue protégées par le capuchon.

De toute façon, l'invention sera bien comprise à l'aide de la description qui suit, en référence au dessin schématique annexé représentant, à titre d'exemples non limitatifs, plusieurs formes d'exécution de cette seringue.
Figure 1 est une vue en coupe longitudinale d'une seringue de l'art antérieur ;
Figures 2, 3 et 4 sont des vues agrandies de la zone d'extrémité comportant l'embout d'une seringue selon l'invention non équipée du capuchon, respectivement de côté, en coupe longitudinale et en bout.
Figures 5, 6 et 7 sont des vues correspondant respectivement aux figures 2, 3 et 4 de la seringue équipée du capuchon ;
Figure 8 est une vue correspondant à la figure 5, la seringue comportant une ceinture d'inviolabilité.
Figure 9 est une vue correspondant à la figure 6, l'obturateur ayant été retiré de l'embout par sectionnement ou déchirement.

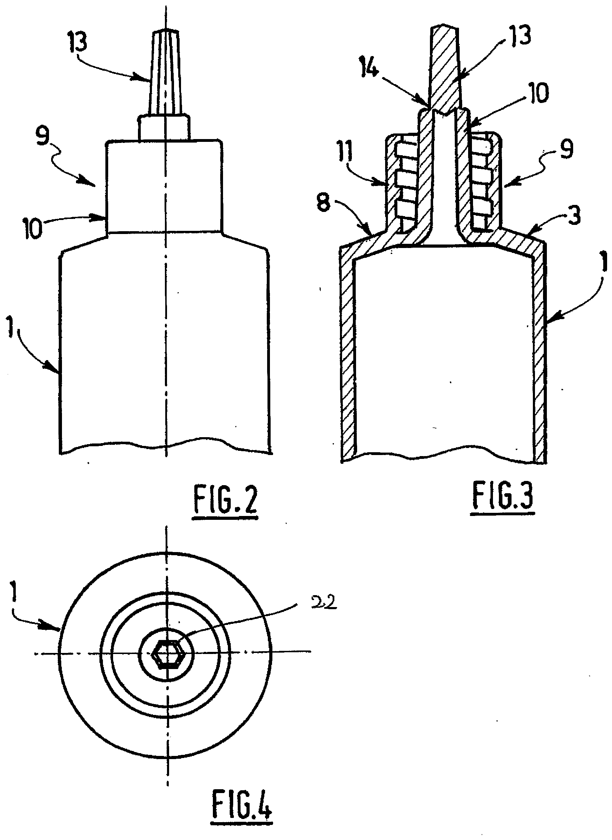

Une seringue hypodermique l'invention est décrite aux figures 2 à 9 dans lesquelles les mêmes éléments sont désignés par les mêmes références qu'à la figure 1.

Les figures 2 et 3 représentent une seringue hypodermique comprenant, comme cela est connu en soi et décrit précédemment, un corps tubulaire 1 comportant une extrémité ouverte 3 au niveau de laquelle est disposé un embout 9 de type Luer-Lock.

L'embout 9 comporte une partie tubulaire 10 communiquant avec l'ouverture de la paroi 8 et un obturateur 13 relié par une zone sécable à l'extrémité libre de la partie tubulaire 10.

Le corps tubulaire, l'embout et l'obturateur sont réalisés en matière synthétique et en une seule pièce par moulage.

La zone sécable 14 entre la partie tubulaire 10 et l'obturateur 13 est réalisée par un amincissement annulaire de la matière le long de la ligne de raccordement entre l'extrémité libre de la partie tubulaire 10 et l'obturateur 13. Cela permet de pouvoir séparer ces deux parties, la zone d'amincissement présentant une faible résistance au déchirement ou à la rupture, sous l'effet d'une torsion.

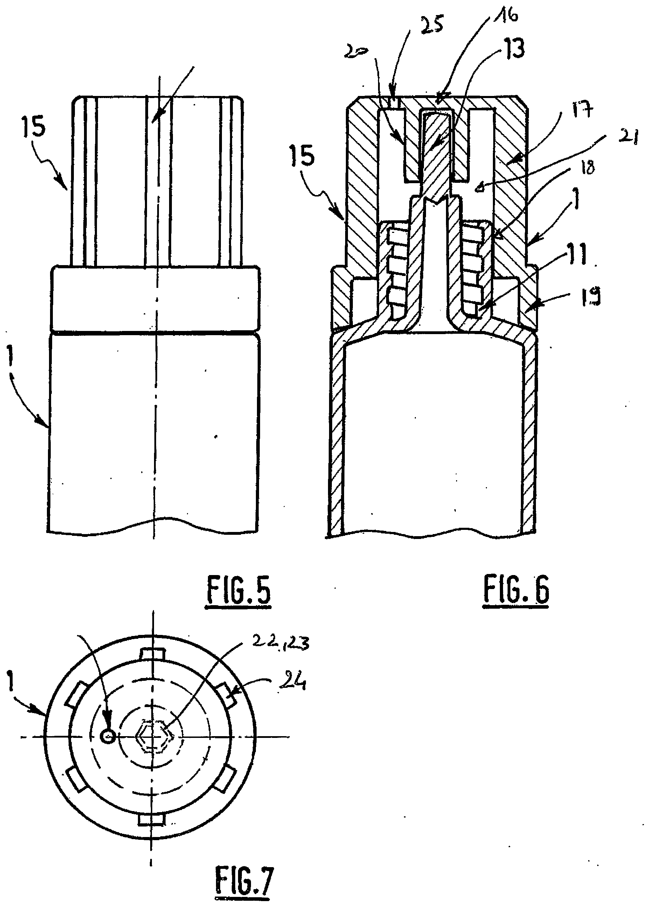

Comme représenté aux figures 5 à 9, un capuchon 15 est monté sur l'embout afin de faciliter les conditions de retrait de l'obturateur 13 tout en préservant la stérilité.

Le capuchon 15 est de forme générale cylindrique. Celui-ci comporte une paroi d'extrémité 16 à partir de laquelle fait saillie une jupe 17, destinée à venir s'engager sur la paroi externe 18 du col formé par la partie filetée 11. Le diamètre interne de la jupe 17 est ajusté de manière à permettre un léger serrage de la jupe 17 sur le col 11.

La jupe 17 s'étend jusqu'au niveau du corps 1 de la seringue, l'extrémité libre 19 de la jupe 17, située du côté du corps 1 de la seringue, ayant sensiblement le même diamètre extérieur que le corps 1. Ceci permet d'assurer une continuité de la surface au niveau de la liaison entre le corps tubulaire 1 de la seringue et la jupe 17 du capuchon 15.

Le capuchon 15 comporte en outre une cheminée cylindrique 20 faisant saillie depuis la paroi d'extrémité 16 du capuchon 15, dans le volume interne 21 du capuchon 15 délimité par la jupe 17.

La cheminée 20 est centrée sur l'axe longitudinal du capuchon et engagée sur l'obturateur 13.

Comme représenté en figure 4 et 7, l'obturateur 13 et la cheminée 20 présentent des formes complémentaires polygonales 22, 23, par exemple hexagonales. En outre, ces deux parties 13, 20 présentent des formes légèrement coniques de façon à assurer un emmanchement à force de l'obturateur 13 dans la cheminée 20.

Selon une autre variante non représentée de l'invention, la cheminée 20 est fixée à l'obturateur 13 par collage ou par soudage.

Le capuchon 15 présente également des cannelures 24 disposées sur la face externe de celui-ci, celles-ci formant des moyens ergonomiques de mise en rotation.

La rotation du capuchon 15 autour de son axe longitudinal entraîne en rotation l'obturateur 13, ce qui provoque la rupture de la zone sécable 14.

Le capuchon 15 est ensuite retiré avec l'obturateur 13, ce dernier étant retenu dans la chemise du fait de l'emmanchement à force, de manière à libérer la partie tubulaire 10 de l'embout 9 pour y raccorder l'aiguille ou la ligne menant au patient.

Le capuchon comporte en outre un orifice 25 destiné au passage du fluide de stérilisation. Lors de la stérilisation en chaleur humide, la vapeur peut ainsi parvenir depuis l'extérieur du capuchon 15 vers les zones à stériliser de l'embout 9.

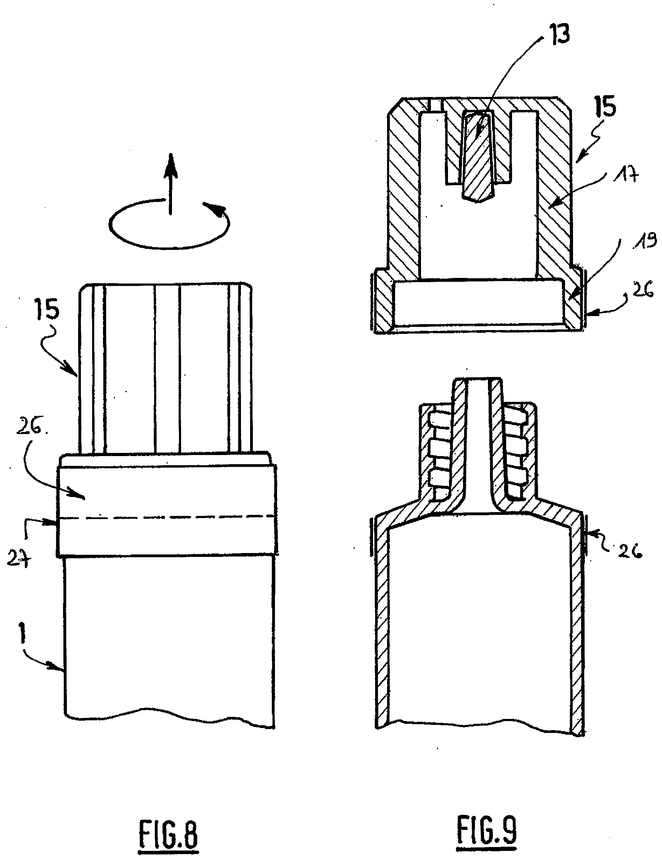

Comme cela est décrit aux figures 8 et 9, une ceinture d'inviolabilité 26 est montée sur la périphérie du corps tubulaire 1 et de la jupe 17 du capuchon 15 et s'étend pour partie sur le corps tubulaire 1 et pour partie sur la jupe 17 au niveau de son extrémité libre 19. La ceinture d'inviolabilité comporte une zone périphérique de rupture 27 située le long de la ligne de raccordement entre la capuchon 15 et le corps tubulaire 1.

La rotation du capuchon 15 permet de sectionner à la fois la ceinture d'inviolabilité 26 et la zone de liaison 14 entre l'obturateur 13 et la partie tubulaire 10 de l'embout 9.

Comme il va de soi, l'invention ne se limite pas aux seules formes d'exécution de ce système, décrites ci-dessus à titre d'exemple, mais elle embrasse au contraire toutes les variantes. C'est ainsi que la cheminée et l'obturateur pourraient présenter d'autres types de formes permettant d'assurer un couplage en rotation de ces deux parties, telles que des cannelures et des rainures complémentaires.

## Revendications

1. Seringue hypodermique préremplie comportant :
- un corps tubulaire (1) comprenant deux extrémités ouvertes (2, 3) et contenant un fluide à administrer à un patient,
- un poussoir (6) qui, comportant à l'une de ses extrémités un piston (4) monté coulissant dans le corps tubulaire (1), traverse l'ouverture de la première extrémité (2) du corps tubulaire,
- un embout (9) de raccordement, par exemple à une aiguille ou à une ligne de perfusion, destiné au passage du fluide à administrer, l'embout (9) comportant une partie tubulaire (10) en forme de cône communiquant avec l'ouverture de la seconde extrémité (3), et un obturateur (13) relié par une zone sécable (14) à l'extrémité libre de la partie tubulaire (10), et
- un capuchon (15) monté sur l'embout (9), le capuchon (15) comportant une zone de fixation ou d'accouplement à l'obturateur (13) de sorte que la rotation du capuchon (15) autour de son axe longitudinal entraîne en rotation l'obturateur (13) de manière à provoquer la rupture de la zone sécable (14),
la seringue étant réalisée en matière synthétique et obtenue par moulage, l'obturateur (13) venant de moulage en une seule pièce avec l'embout (9) et le corps tubulaire (10),
**caractérisé en ce que** l'embout (9) de raccordement est du type Luer-Lock et comprend une partie filetée (11) entourant la partie tubulaire (10), **en ce que** le capuchon (15) comporte :
- une jupe (17) engagée sur la paroi externe (18) de la partie filetée (11) et s'étendant jusqu'au niveau du corps (1) de la seringue, la partie (19) de la jupe (17) située du côté du corps (1) de la seringue ayant, sur au moins une partie de sa longueur, sensiblement la même section que le corps (1) de la seringue, et
- au moins un orifice (25) destiné au passage d'un fluide de stérilisation, et
**en ce que** la seringue comporte une ceinture d'inviolabilité (26) s'étendant pour partie sur le corps (1) de la seringue et pour partie sur la jupe (17, 19) du capuchon (15).

2. Seringue hypodermique selon la revendication 1, **caractérisée en ce que** la liaison entre la partie tubulaire (10) de l'embout (9) et l'obturateur (13) est réalisée par un amincissement annulaire (14) de la matière le long de la ligne de raccordement entre l'extrémité libre de la partie tubulaire (10) de l'embout (9) et l'obturateur (13).

3. Seringue hypodermique selon l'une des revendications 1 et 2, **caractérisée en ce que** le capuchon (15) comporte une paroi d'extrémité (16) à partir de laquelle fait saillie une cheminée (20) qui, engagée sur l'obturateur (13), coopère avec celui-ci par complémentarité de forme de manière à réaliser un couplage en rotation.

4. Seringue hypodermique selon la revendication 3, **caractérisée en ce que** l'obturateur (13) et la cheminée (20) présentent des formes polygonales complémentaires, par exemple hexagonales.

5. Seringue hypodermique selon l'une des revendications 3 et 4, **caractérisé en ce que** l'obturateur (13) et/ou la cheminée (20) présentent une forme conique.

6. Seringue hypodermique selon l'une des revendications 1 et 2, **caractérisée en ce que** le capuchon (15) est fixé sur l'obturateur (13) par soudage ou par collage.

7. Seringue hypodermique selon l'une des revendications 1 à 6, **caractérisé en ce que** le capuchon (15) comporte des moyens ergonomiques de mise en rotation (24).

## Patentansprüche

1. Vorgefüllte subkutane Injektionsspritze, die Folgendes umfasst:
- einen röhrenförmigen Körper (1), der zwei offene Enden (2, 3) umfasst und ein Fluid enthält, das einem Patienten zu verabreichen ist,
- einen Drücker (6), der an einem seiner Enden einen Kolben (4) umfasst, der gleitend in dem röhrenförmigen Körper (1) montiert ist und die Öffnung des ersten Endes (2) des röhrenförmigen Körpers durchquert,
- einen Anschlussansatz (9), zum Beispiel einer Nadel oder einer Infusionsleitung, der zum Durchgehen des zu verabreichenden Fluids bestimmt ist, wobei der Ansatz (9) einen röhrenförmigen Teil (10) in Kegelform umfasst, der mit der Öffnung des zweiten Endes (3) kommuniziert, und einen Verschluss (13), der durch eine trennbare Zone (14) mit dem freien Ende des röhrenförmigen Teils (10) verbunden ist, und
- eine Kappe (15), die auf den Ansatz (9) montiert ist, wobei die Kappe (15) eine Befestigungs- oder Kopplungszone mit dem Verschluss (13) derart umfasst, dass die Drehung der Kappe (15) um ihre Längsachse den Verschluss (13) derart in Drehung antreibt, dass der Bruch der trennbaren Zone (14) verursacht wird,
wobei die Spritze aus einem Kunststoff hergestellt und durch Formen erhalten wird, wobei der Verschluss (13) in einem Stück mit dem Ansatz (9) und dem röhrenförmigen Körper (10) geformt ist,
**dadurch gekennzeichnet, dass** der Anschlussansatz (9) des Typs Luer-Lock ist und einen Gewindeteil (11) umfasst, der den röhrenförmigen Teil (10) umgibt, und dadurch, dass die Kappe (15) Folgendes umfasst:
- eine Schürze (17), die auf der Außenwand (18) des Gewindeteils (11) eingreift und sich bis zu dem Bereich des Körpers (1) der Spritze erstreckt, wobei der Teil (19) der Schürze (17), der auf der Seite des Körpers (1) der Spritze liegt, auf mindestens einem Teil seiner Länge im Wesentlichen denselben Querschnitt hat wie der Körper (1) der Spritze, und
- mindestens eine Öffnung (25), die für das Durchgehen eines Sterilisationsfluids bestimmt ist, und
dadurch, dass die Spritze eine Unverletzlichkeitsumgurtung (26) umfasst, die sich zum Teil auf dem Körper (1) der Spritze erstreckt und zum Teil auf der Schürze (17, 19) der Kappe (15).

2. Subkutane Injektionsspritze nach Anspruch 1, **dadurch gekennzeichnet, dass** die Verbindung zwischen dem röhrenförmigen Teil (10) des Ansatzes (9) und dem Verschluss (13) durch eine ringförmige Verjüngung (14) des Materials entlang der Anschlusslinie zwischen dem freien Ende des röhrenförmigen Teils (10) des Ansatzes (9) und dem Verschluss (13) hergestellt ist.

3. Subkutane Injektionsspritze nach einem der Ansprüche 1 und 2, **dadurch gekennzeichnet, dass** die Kappe (15) eine Endwand (16) umfasst, von der ausgehend ein Kamin (20) vorragt, der auf dem Verschluss (13) eingreifend mit diesem durch Formkomplementarität derart zusammenwirkt, dass eine Drehungskopplung hergestellt wird.

4. Subkutane Injektionsspritze nach Anspruch 3, **dadurch gekennzeichnet, dass** der Verschluss (13) und der Kamin (20) komplementäre polygonale Formen, die zum Beispiel sechseckig sind, aufweisen.

5. Subkutane Injektionsspritze nach einem der Ansprüche 3 und 4, **dadurch gekennzeichnet, dass** der Verschluss (13) und/oder der Kamin (20) eine Kegelform aufweisen.

6. Subkutane Injektionsspritze nach einem der Ansprüche 1 und 2, **dadurch gekennzeichnet, dass** die Kappe (15) auf dem Verschluss (13) durch Schweißen oder durch Kleben befestigt ist.

7. Subkutane Injektionsspritze nach einem der Ansprüche 1 bis 6, **dadurch gekennzeichnet, dass** die Kappe (15) ergonomische Mittel zum Indrehungversetzen (24) umfasst.

## Claims

1. A pre-filled hypodermic syringe comprising:
- a tubular body (1) having two open ends (2, 3) and containing a fluid to be administered to a patient,
- a plunger (6) which, at one of its ends, has a piston (4) mounted slidably in the tubular body (1) and which passes through the opening of the first end (2) of the tubular body,
- a connection tip (9), for example for connection to a needle or to an infusion line, intended for the passage of the fluid that is to be administered, the tip (9) comprising a tubular part (10) in the form of a cone communicating with the opening of the second end (3), and an obturator (13) connected by a frangible zone (14) to the free end of the tubular part (10), and
- a cap (15) mounted on the tip (9), the cap (15) having a zone of fixation or coupling to the obturator (13) such that the rotation of the cap (15) about its longitudinal axis moves the obturator (13) in rotation in such a way as to cause the frangible zone (14) to break,
the syringe being made of synthetic material and produced by molding, the obturator (13) being molded in one piece with the tip (9) and the tubular body (10),
**characterized in that** the connection tip (9) is of the Luer-lock type and includes a threaded part (11) surrounding the tubular part (10),
**in that** the cap (15) has:
- a skirt (17) engaged on the outer wall (18) of the threaded part (11) and extending as far as the body (1) of the syringe, the part (19) of the skirt situated toward the body (1) of the syringe having, over at least part of its length, substantially the same cross section as the body (1) of the syringe, and
- at least one orifice (25) intended for passage of a sterilization fluid, and
**in that** the syringe includes a tamper-proof ring (26) extending partly on the body (1) of the syringe and partly on the skirt (17, 19) of the cap (15).

2. The hypodermic syringe as claimed in claim 1, **characterized in that** the join between the tubular part (10) of the tip (9) and the obturator (13) is formed by an annular thinning (14) of the material along the line of connection between the free end of the tubular part (10) of the tip (9) and the obturator (13).

3. The hypodermic syringe as claimed in one of claims 1 and 2, **characterized in that** the cap (15) has an end wall (16) from which protrudes a hollow stub (20) which, when engaged on the obturator (13), cooperates with the latter through having a matching shape, in such a way as to form a rotation coupling

4. The hypodermic syringe as claimed in claim 3, **characterized in that** the obturator (13) and the hollow stub (20) have matching polygonal shapes, for example hexagonal shapes.

5. The hypodermic syringe as claimed in one of claims 3 and 4, **characterized in that** the obturator (13) and/or the hollow stub (20) have a conical shape.

6. The hypodermic syringe as claimed in one of claims 1 and 2, **characterized in that** the cap (15) is fixed on the obturator (13) by welding or adhesive bonding.

7. The hypodermic syringe as claimed in one of claims 1 through 6, **characterized in that** the cap (15) has ergonomic means (24) for rotating it.
